# EUROPÄISCHE PATENTSCHRIFT

(11) **EP 1 709 172 B1**
(45) Veröffentlichungstag und Bekanntmachung des Hinweises auf die Patenterteilung: **26.09.2007**
(21) Anmeldenummer: 04797840.8
(22) Anmeldetag: 12.11.2004
(51) Int. Cl.: C12N 15/10, C12Q 1/68

(54) **VERFAHREN UND KIT ZUR ISOLIERUNG VON RNA**
METHOD AND KIT FOR ISOLATING RNA
METHODE ET KIT D'ISOLATION D'ARN

(30) Priorität: 12.12.2003 DE 10358137
(43) Veröffentlichungstag der Anmeldung: 11.10.2006
(73) Patentinhaber: Merck Patent GmbH, 64293 Darmstadt (DE)
(72) Erfinder: MICHELSEN, Uwe, 69469 Weinheim (DE); HOLSCHUH, Karl, 64342 Seeheim-Jugenheim (DE); SCHWÄMMLE, Achim, 64283 Darmstadt (DE)
(86) Internationale Anmeldenummer: PCT/EP2004/012819
(87) Internationale Veröffentlichungsnummer: WO 2005/061708

(56) Entgegenhaltungen:
- EP-A- 0 389 063
- EP-A- 1 002 860
- WO-A-00/70040
- WO-A-98/31840
- DAVIES MARTIN J ET AL: "Isolation of plasmid DNA using magnetite as a solid-phase adsorbent" ANALYTICAL BIOCHEMISTRY, Bd. 262, Nr. 1, 15. August 1998 (1998-08-15), Seiten 92-94, XP002321531 ISSN: 0003-2697

## Beschreibung

Die Erfindung betrifft ein Verfahren und einen Kit zur Isolierung von RNA in Anwesenheit von DNA durch spezifische Bindung an Magnetit-Träger.

Die Anreicherung bzw. Reinigung und Isolierung von verschiedenen Nukleisäurespezies wie doppelsträngiger Plasmid DNA, chromosomaler DNA, einzelsträngiger DNA, DNA Fragmenten oder RNA ist von zentraler Bedeutung in der Molekularbiologie. Seit langem ist eine Vielzahl von Methoden bekannt, um die verschiedenen DNA Spezies, die in einer Probe vorkommen können, voneinander zu trennen. Hierbei kommen Verfahren auf rein flüssig-chemischer Basis zum Einsatz oder auch verschiedene Verfahren mit Hilfe von spezifisch modifizierten Festphasen zur Bindung der Nukleinsäuren.

Besonderes Interesse hat in den letzten Jahren die Analyse der RNA erfahren, da insbesondere die RNA Moleküle durch ihre vielfältigen Funktionen den biologischen Zustand einer Zelle widerspiegeln. Zum Anderen sind bedeutende pathogene Viren mit RNA Genomen ausgestattet, die es mittels der molekularen Diagnostik quantitativ und qualitativ nachzuweisen gilt.

Ein rein flüssig-chemisches Verfahren für die spezifische Isolierung von RNA Molekülen beschreibt US 4,843,155. Die Methode macht sich das unterschiedliche Lösungsverhalten von DNA und RNA zunutze. Jedoch kommen hierbei hochgiftige Substanzen wie Phenol oder Chloroform zum Einsatz. Zudem müssen zeitaufwendige Fällungsreaktionen mit Alkohol und Zentrifugationen durchgeführt werden.

US 5,234,809 und US 6,355,792 beschreiben den Einsatz einer Festphase zur Isolierung von DNA und RNA. Eine Unterscheidung zwischen den beiden Nukleinsäure-Spezies ist nicht möglich.

WO 92/18514 und WO 01/46404 beschreiben ähnliche Verfahren zur Isolierung von DNA/RNA, bei denen metallische Oxide wie z.B. Magnetit als Festphase eingesetzt werden.

Es wurde nun gefunden, dass unmodifizierte Magnetit-Partikel unter bestimmten chaotropen Bedingungen spezifisch und effektiv RNA-Moleküle binden, wohingegen DNA-Moleküle im Überstand verbleiben. Dies ist insbesondere überraschend, da in der Literatur (M. J. Davies et al., Analytical Biochemistry 262, 92-94 (1998), WO 92/18514 und WO 01/46404) ebensolche Magnetit-Träger zur Isolierung von DNA verwendet werden.

Gegenstand der vorliegenden Erfindung ist daher ein Verfahren zur Isolierung von RNA aus Proben gekennzeichnet durch folgende Verfahrensschritte:
a) Bereitstellung einer Festphase aus Magnetit
b) Bereitstellung eines Bindepuffers, der Guanidiniumthiocyanat (GTC) in einer Konzentration enthält, die nach Mischung mit der Probe eine Endkonzentration von > 2,5 M Guanidiniumthiocyanat bewirkt;
c) Herstellung einer Mischung aus der Probe, der Festphase aus Magnetit und dem Bindepuffer, wobei in dieser Mischung eine die Bindung der RNA unterstützende Phosphat-Konzentration vorliegt;
d) Isolierung der Festphase mit der gebundenen RNA

In einer bevorzugten Ausführungsform wird im Anschluß an die Isolierung der Festphase mit der gebundenen RNA (Schritt d))
e) die besagte Festphase optional gewaschen und
f) die RNA von der Festphase eluiert.

In einer bevorzugten Ausführungsform erfolgt die Elution in Schritt e) mit Elutionspuffern, die einen pH-Bereich > 7 ermöglichen und Phosphat enthalten.

In einer weiteren bevorzugten Ausführungsform enthält der Bindepuffer zusätzlich Chelatoren wie EDTA.

In einer bevorzugten Ausführungsform besteht die Festphase aus partikulärem Magnetit. Besonders bevorzugt sind Magnetit-Partikel mit einem Durchmesser von 0,01 bis 2 µm und einer spezifischen Oberfläche von 1 bis 100 m²/g.

Gegenstand der vorliegenden Erfindung ist auch ein Kit zur Isolierung von RNA nach dem erfindungsgemäßen Verfahren zumindest enthaltend eine Festphase aus Magnetit und einen Bindepuffer mit einer GTC-Konzentration von über 3 Mol/l.

In einer bevorzugten Ausführungsform enthält der Bindepuffer zumindest zwischen 4 und 8 Mol/l GTC, zwischen 5 und 200 mMol/l EDTA und typischerweise zwischen 10 und 100 mMol/l Tris-HCl oder ähnliche Puffersubstanzen.

In einer bevorzugten Ausführungsform enthält der Kit zusätzlich einen oder mehrere der folgenden Bestandteile
- einen Elutionspuffer
- einen Waschpuffer
- eine Phosphatsalzlösung

Proben im Sinne der Erfindung sind jede Art von Proben, in denen RNA vermutet wird. Die Probe kann synthetischen oder bevorzugt gentechnologischen, biotechnologischen oder biologischen Ursprungs sein, d.h. z.B. aus Bakterien, Viren, Körperzellen, Blut, Plasma,
Zerebrospinalflüssigkeit, Harn, Feces, Milch, Gewebe, Fermentationsbrühe oder Zellkulturen.
Die Probe kann direkt eingesetzt werden oder falls notwendig zuerst aufgeschlossen werden. Beispielsweise müssen Proben, die Zellen oder virale Partikel enthalten zunächst aufgeschlossen werden, um die RNA aus den Zellen bzw. Partikeln freizusetzen. Geeignete Methoden zum Aufschluß der Proben bzw. Lyse von Zellen sind dem Fachmann bekannt.

RNA ist jede natürliche oder synthetische Form der Ribonukleinsäure, insbesondere m-RNA, t-RNA, ribosomale RNA, virale RNA oder synthetische RNA Moleküle wie siRNA (RNAi).

Der Begriff "Phosphat" umschliesst erfindungsgemäß anorganisches Phosphat und organisches Phosphat. Beispiele hierfür sind Phosphatsalze wie Natriumhydrogenphosphat (anorganisches Phosphat) oder phosphathaltige Kohlenwasserstoffverbindungen wie Aminosäuren, Kreatinphosphat und phosphathaltige Proteine (organisches Phosphat).

Eine Festphase aus Magnetit ist ein Träger, dessen Oberfläche zumindest größtenteils, bevorzugt vollständig, aus Magnetit (Fe₃O₄) besteht. Die Festphase kann beispielsweise als Platte, Partikel, Beschichtung, Faser, Filter oder andere bevorzugt poröse Struktur vorliegen. Besonders bevorzugt besteht die Festphase aus Magnetit-Partikeln.
Zur Herstellung von Magnetit-Partikeln sind verschiedene Herstellverfahren bekannt. Beispiele sind offenbart in:
- Massart, IEEE Trans. Magn. 17, 1247-1248 (1981)
- Sugimoto, Matijevic, J. Colloid Interface Sci. 74, 227-243 (1980)
- Qu et al., J. Colloid Interface Sci. 215,190-192 (1999)
Besonders bevorzugt erfolgt die Herstellung der Festphase aus Magnetit nach Sugimoto und Matijevic.

In der Regel bindet 1 mg derartiger Partikel ca. 10 µg RNA. Da die meisten Proben weniger als 1 µg RNA enthalten, werden typischerweise für die erfindungsgemäße Isolierung von RNA 0,5 bis 5 mg, bevorzugt ca. 1 mg, Partikel eingesetzt.

Kern der vorliegenden Erfindung ist, dass RNA in wässrigen Lösungen in Anwesenheit von Guanidiniumthiocyanat (GTC) und Phosphat an Festphasen aus Magnetit bindet, während DNA in Lösung bleibt. Auf diese Weise können in der Regel mehr als 70%, häufig mehr als 90% der in der Probe vorhandenen RNA spezifisch isoliert werden.

Um die Bindung der RNA zu gewährleisten muß der wässrige Bindepuffer bei der Mischung mit der Probe und der Festphase eine Konzentration von mindestens 2,5 M GTC, bevorzugt 3-5 M GTC, ermöglichen. Dazu werden je nach dem Volumenverhältnis der Probe und des Bindepuffers bevorzugt Bindepuffer mit einer Konzentration von über 3 M, bevorzugt 5 bis 8 M GTC eingesetzt.

Die spezifische Bindung der RNA findet unter geeigneten Bedingungen in einem breiten pH Bereich statt. Bevorzugt stellt der Bindungspuffer einen pH-Bereich von 6 - 10 ein, besonders bevorzugt von pH 7 - 9. Daher enthält der Bindepuffer Puffersubstanzen, die dies ermöglichen. Dem Fachmann sind geeignete Puffersubstanzen bekannt. Beispiele hierfür sind Tris-HCl, Tricine, MOPS und andere.

Essentiell für die spezifische Isolierung der RNA ist das Vorhandensein einer bestimmten Menge an Phosphat während der Bindung an die Magnetit-Festphase.
Liegt während der Bindung (d.h. bei Mischung der Probe mit dem Bindepuffer und der Festphase) kein Phosphat vor, binden in der Regel sowohl DNA als auch RNA an das Magnetit. Liegt während der Bindung zuviel Phosphat vor, so binden weder DNA noch RNA.

Die Menge an Phosphat, die zur Unterstützung der spezifischen Bindung der RNA notwendig ist, hängt von der Größe der Oberfläche der Festphase aus Magnetit ab. Wahrscheinlich ist der Grund für diesen Zusammenhang eine Wechselwirkung zwischen der Oberfläche der Festphase und dem Phosphat. Die Menge an Phosphat muss ausreichend groß sein, um die Bindung der DNA an die Oberfläche der Festphase zu verhindern, darf aber nicht so groß sein, dass das Phosphat die Bindung der RNA stört.

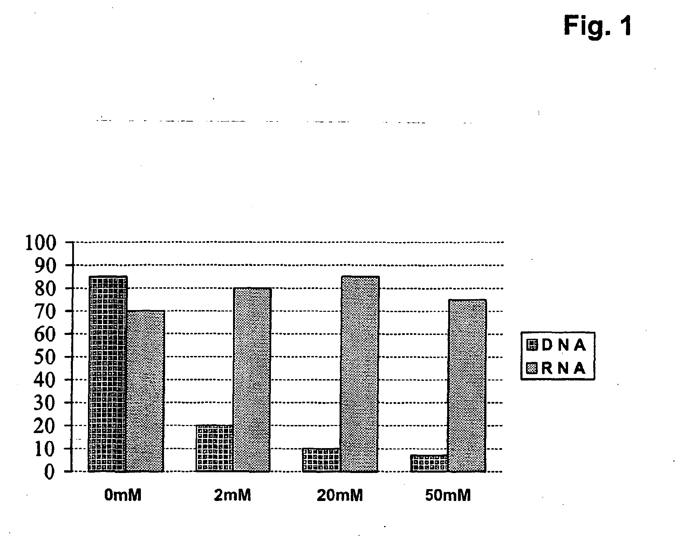

Wie beispielhaft in Abbildung 1 dargestellt, bewirkt eine Konzentration von 20mM Phosphat pro mg der bevorzugt eingesetzten Magnetit-Partikel (Merck MagPrep^{®} Magnetit, Art.Nr. 101882, d.h. Partikel mit Durchmesser ca. 1 µm, spezifischer Oberfläche ca. 20 m²/g, hergestellt nach Sugimoto, Matijevic, J. Colloid Interface Sci. 74, 227-243 (1980)) eine spezifische und effiziente Bindung der RNA an die Festphase aus Magnetit. Die DNA verbleibt in Lösung.
Für eine effiziente Bindung der RNA sollte die Phosphat-Konzentration nicht größer sein als 200mM pro mg der benutzten Magnetit-Partikel.

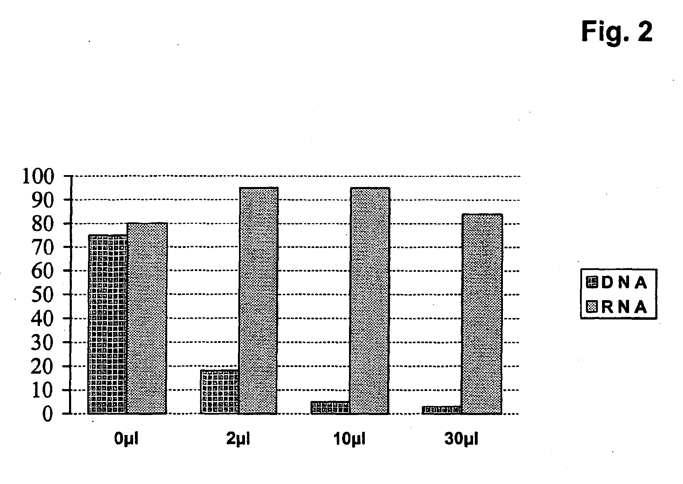

Die zur Unterstützung der spezifischen Bindung der RNA notwendige Menge an Phosphat kann dem Gemisch durch entsprechende Zusätze zum Bindepuffer zugesetzt werden. Genauso kann jedoch auch die Probe selbst bereits Phosphat enthalten, wie es für Körperflüssigkeiten wie Urin oder Blutplasma, das Phosphat in anorganischer und in Protein-gebundener Form enthält, bekannt ist. In diesem Falle kann durch das Mengenverhältnis von Probe und Bindepuffer und/oder der Menge an Magnetit die notwendige Phosphatmenge für die spezifische RNA Bindung eingestellt werden. Dies ist beispielhaft in Abbildung 2 dargestellt.

Somit bedeutet die Angabe, dass die Mischung aus Bindepuffer, wässriger Probe und Festphase eine "die spezifische Bindung der RNA unterstützende" Phosphat-Konzentration enthält erfindungsgemäß, dass die Mischung soviel anorganisches und/oder organisches Phosphat enthält, dass eine Bindung der DNA verhindert und eine Bindung der RNA unterstützt wird.

Bei Proben biologischen Ursprungs müssen die Nukleinsäure-haltigen Zellen oder Viren lysiert werden, um die RNA gemäß des erfindungsgemäßen Verfahrens quantitativ anzureichern. Zur Unterstützung der Lyse benützt der Fachmann unter anderem nicht-ionische Detergenzien, wie NP 40, Tween^{®} 20 oder Triton^{®} X-100. Diese nichtionischen Detergenzien können die Effizienz der erfindungsgemäßen RNA-Isolierung negativ beeinflussen. Es wurde jedoch gefunden, dass dieser negative Einfluss durch einen Zusatz von Chelatoren wie EDTA aufgehoben wird.

Daher enthält der Bindepuffer und/oder die Probe erfindungsgemäß bevorzugt zusätzlich Chelatoren wie EDTA. Bevorzugt liegt die Konzentration an Chelatoren während der Bindung zwischen 5 und 200 mMol/l. Daher enthält der Bindepuffer typischerweise zwischen 5 und 200 mMol/l, bevorzugt zwischen 10 und 100 mMol/l Chelatoren.
Zudem kann der Bindepuffer weitere Substanzen wie Stabilisatoren, Enzyminhibitoren etc. enthalten.

Zur Durchführung des erfindungsgemäßen Verfahrens wird die Probe typischerweise zunächst mit dem Bindepuffer gemischt und dann mit der Festphase versetzt. Die Suspension aus Probe, Bindepuffer und Festphase wird gut durchmischt.
Nach einer Inkubationszeit von 3 bis 60 Minuten, typischerweise ca. 10 Minuten, wird der Überstand sorgfältig abgetrennt.

Die Festphase wird optional z.B. mit einem sauren Waschpuffer, wie er beispielsweise in US 6,355,792 offenbart ist, gewaschen.
Falls eine Isolierung der RNA gewünscht wird, wird nach erneuter Separation und Entfernung des Überstands die Festphase im Elutionspuffer resuspendiert, gut durchmischt und 3 bis 60, typischerweise 10 Minuten, inkubiert. Nach Abtrennen der Festphase kann der Überstand mit der RNA zur weiteren Analyse verwendet werden.

Gegenstand der vorliegenden Erfindung ist auch ein Kit zur Isolierung von RNA mittels des erfindungsgemäßen Verfahrens. Der Kit enthält eine Festphase aus Magnetit und einen Bindepuffer mit einer Konzentration von mindestens 3 Mol/l GTC. Weiterhin enthält der Kit bevorzugt zusätzlich Waschpuffer und Elutionspuffer sowie eine Phosphatsalzlösung für das Einstellen des notwendigen Phosphatgehaltes einer Probe.
In einer bevorzugten Ausführungsform enthält der Bindepuffer zumindest GTC in einer Konzentration zwischen 4 und 8 Mol/l, EDTA in einer Konzentration zwischen 5 und 200 mMol/l und als Puffersubstanz typischerweise Tris-HCl in einer Konzentration zwischen 10 und 100 mMol/l.

In einer bevorzugten Ausführungsform enthält der Kit als Festphase Magnetit-Partikel hergestellt nach Sugimoto, Matijevic, J. Colloid Interface Sci. 74, 227-243 (1980).

Somit liefern das erfindungsgemäße Verfahren sowie der erfindungsgemäße Kit erstmals eine effektive und quantitative Möglichkeit zur Festphasen-unterstützten Isolierung von RNA aus Proben in Anwesenheit von DNA.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen, insbesondere der korrespondierenden Anmeldung DE 10358137.5, eingereicht am 12.12.2003, ist durch Bezugnahme in diese Anmeldung eingeführt.

### Beispiele

### Beispiel 1

1 mg MagPrep^{®} Magnetit Partikel (Merck KGaA) werden mit einem Mix von 500µl Bindepuffer B (5M Guanidinium Thiocyanat + 50mM Tris-HCl pH 7,5 + 20mM EDTA + 1% Triton^{®}X 100), DNA (200ng linearisierte Plasmid-DNA) und RNA (200ng 16S/23S-RNA) gemischt. Der Mix wurde zuvor mit steigenden Mengen Natriumphosphat versetzt.
Nach 10 Minuten Inkubation wird magnetisiert und der Überstand sorgfältig abgetrennt. Nach Entfernung des Magnetfeldes werden die Partikel mit 500µl Waschpuffer AT (10mM Acetat-Tris pH 4.0) resuspendiert und bis zu 10 Minuten bei Raumtemperatur inkubiert.
Nach Magnetisierung wird der Überstand sorgfältig entfernt und wieder mit 500µl Waschpuffer AT versetzt. Nach Entfernung des Magnetfeldes werden die Partikel resuspendiert, magnetisiert und der Überstand verworfen. Die Elution der Nukleinsäuren von den Partikeln erfolgt nach 10-minütiger Inkubation in 100µl Elutionspuffer P (10mM Tris-HCl pH 8,5 +1mM EDTA + 50mM Natriumhdrogenphosphat) bei 50°C. Nach Magnetisierung wird das Eluat in ein neues, steriles Gefäß transferiert.
Die Konzentration der DNA und RNA wird durch eine Fluoreszenzmessung mit PicoGreen^{®} und RiboGreen^{®} (Molecular Probes) gemäß den Angaben des Herstellers quantifiziert. Die Abbildung 1 zeigt die Ausbeute an DNA und RNA in Abhängigkeit zur Phosphat-Konzentration. Auf der Abszisse ist die Phosphatkonzentration, auf der Ordinate die Wiederfindungsrate von DNA und RNA in % angegeben.

Es ist klar ersichtlich, dass die Bindung der DNA schon bei Anwesenheit geringer Mengen an Phosphat stark abnimmt. Dagegen bindet die RNA bei 2 bis 50 mM Phosphat effizient.

### Beispiel 2

1 mg MagPrep^{®} Magnetit Partikel (Merck KGaA) werden mit einem Mix von 500µl Bindepuffer B (5M Guanidinium Thiocyanat + 50mM Tris-HCl pH 7,5 + 20mM EDTA + 1% Triton^{®}X 100), DNA (200ng linearisierte Plasmid-DNA) und RNA (200ng 16S/23S-RNA) gemischt. Der Mix wurde zuvor mit steigenden Mengen Human-Plasma versetzt.
Die weiteren Schritte sind in Beispiel 1 beschrieben.
Die Abbildung 2 zeigt die Ausbeute an DNA und RNA in Abhängigkeit zur Plasmakonzentration. Auf der Abszisse ist die Menge an zugegebenem Plasma, auf der Ordinate die Wiederfindungsrate in % angegeben.

Da Plasma phosphorylierte Proteine und anorganisches Phosphat enthält, zeigt sich bei Zusatz von Plasma das gleiche Bild wie bei Zusatz von anorganischem Phosphat (Beispiel 1). Dies zeigt, dass in vielen Fällen, in denen die Probe bereits anorganisches und/oder organisches Phosphat enthält, ein weiterer Zusatz von Phosphatsalzen nicht notwendig ist.

### Beispiel 3

1mg MagPrep^{®} Magnetit Partikel (Merck KGaA) werden mit 500µl von vier verschiedenen Bindepuffern
5M Guanidinium Thiocyanat, (GTC) oder
5M Guanidinium-HCl, (GHCl) ) oder
5M Natrium Thiocyanat, (NaTC) oder
5M Natriumperchlorat, (NaClO)
gemischt. Alle Bindepuffer enthalten DNA (200ng linearisierte Plasmid-DNA) und RNA (200ng 16S/23S-RNA) sowie 3mg/ml Rinderserumalbumin. Die weiteren Schritte sind in Beispiel 1 beschrieben.

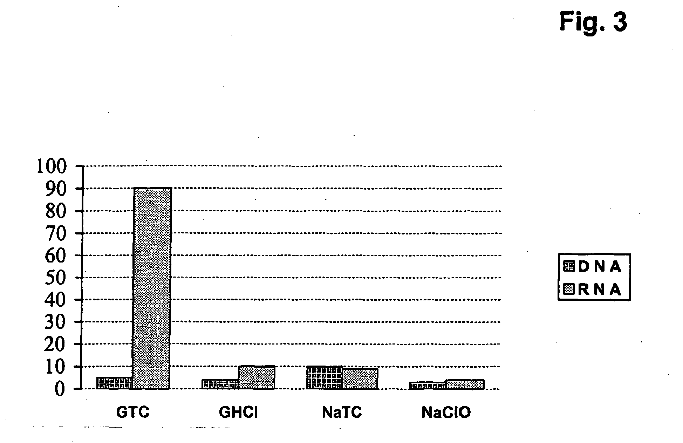

Die Abbildung 3 zeigt die Ausbeute an DNA und RNA in Abhängigkeit von dem eingesetzten chaotropen Salz. Die spezifische Bindung der RNA erfolgt nur in Gegenwart von Guanidiniumthiocyanat.

## Patentansprüche

1. Verfahren zur Isolierung von RNA aus Proben, **gekennzeichnet durch** folgende Verfahrensschritte:
a) Bereitstellung einer Festphase aus Magnetit
b) Bereitstellung eines Bindepuffers, der Guanidiniumthiocyanat in einer Konzentration enthält, die nach Mischung mit der Probe eine Endkonzentration von > 2,5 M Guanidiniumthiocyanat bewirkt;
c) Mischen der Probe mit der Festphase aus Magnetit und dem Bindepuffer, wobei in dieser Mischung eine die Bindung der RNA unterstützende Phosphat-Konzentration vorliegt;
d) Isolierung der Festphase mit der gebundenen RNA.

2. Verfahren nach Anspruch 1, **dadurch gekennzeichnet, dass** im Anschluß an Schritt d) die Festphase optional gewaschen wird und anschließend die RNA von der Festphase eluiert wird.

3. Verfahren nach Anspruch 2, **dadurch gekennzeichnet, dass** die Elution mit Elutionspuffern erfolgt, die einen pH-Bereich > 7 ermöglichen und Phosphat enthalten.

4. Verfahren nach einem oder mehreren der Ansprüche 1 bis 3, **dadurch gekennzeichnet, dass** der Bindepuffer zusätzlich Chelatoren wie EDTA enthält.

5. Verfahren nach einem oder mehreren der Ansprüche 1 bis 4, **dadurch gekennzeichnet, dass** die Festphase aus Magnetit-Partikeln mit einem Durchmesser von 0,01 bis 2 µm und einer spezifischen Oberfläche von 1 - 100 m²/g besteht.

6. Kit zur Isolierung von RNA nach dem Verfahren gemäß einem oder mehreren der Ansprüche 1 bis 5 zumindest enthaltend eine Festphase aus Magnetit und einen Bindepuffer mit einer GTC-Konzentration von über 3 Mol/l.

7. Kit nach Anspruch 6, **dadurch gekennzeichnet, dass** der Bindepuffer zumindest zwischen 4 und 8 Mol/l GTC und zwischen 5 und 200 mMol/l EDTA enthält.

8. Kit nach Anspruch 6 oder 7, **dadurch gekennzeichnet, dass** der Kit zusätzlich einen oder mehrere der folgenden Bestandteile enthält
- einen Elutionspuffer
- einen Waschpuffer
- eine Phosphatsalzlösung.

## Claims

1. Method for the isolation of RNA from samples, **characterised by** the following method steps:
a) provision of a magnetite solid phase;
b) provision of a binding buffer which comprises guanidinium thiocyanate in a concentration which, after mixing with the sample, produces a final concentration of > 2.5M guanidinium thiocyanate;
c) mixing of the sample with the magnetite solid phase and the binding buffer, where a phosphate concentration which supports the binding of RNA is present in this mixture;
d) isolation of the solid phase with the bound RNA.

2. Method according to Claim 1, **characterised in that**, after step d), the solid phase is optionally washed, and the RNA is subsequently eluted from the solid phase.

3. Method according to Claim 2, **characterised in that** the elution is carried out using elution buffers which facilitate a pH range > 7 and comprise phosphate.

4. Method according to one or more of Claims 1 to 3, **characterised in that** the binding buffer additionally comprises chelators, such as EDTA.

5. Method according to one or more of Claims 1 to 4, **characterised in that** the solid phase consists of magnetite particles having a diameter of 0.01 to 2 µm and a specific surface area of 1 - 100 m²/g.

6. Kit for the isolation of RNA by the method according to one or more of Claims 1 to 5, at least comprising a magnetite solid phase and a binding buffer having a GTC concentration of greater than 3 mol/l.

7. Kit according to Claim 6, **characterised in that** the binding buffer comprises at least between 4 and 8 mol/l of GTC and between 5 and 200 mmol/I of EDTA.

8. Kit according to Claim 6 or 7, **characterised in that** the kit additionally comprises one or more of the following constituents:
- an elution buffer
- a wash buffer
- a phosphate salt solution.

## Revendications

1. Procédé pour l'isolation d'ARN à partir d'échantillons, **caractérisé par** les étapes de procédé qui suivent:
a) fourniture d'une phase solide de magnétite;
b) fourniture d'un tampon de liaison qui comprend du thiocyanate de guanidinium selon une concentration qui, après mélange avec l'échantillon, produit une concentration finale > 2,5M de thiocyanate de guanidinium;
c) mélange de l'échantillon avec la phase solide de magnétite et le tampon de liaison, où une concentration en phosphate qui supporte la liaison d'ARN est présente dans ce mélange;
d) isolation de la phase solide avec l'ARN lié.

2. Procédé selon la revendication 1, **caractérisé en ce que**, après l'étape d), la phase solide est en option lavée, et l'ARN est ensuite élué à partir de la phase solide.

3. Procédé selon la revendication 2, **caractérisé en ce que** l'élution est mise en oeuvre en utilisant des tampons d'élution qui facilitent une plage de pH > 7 et qui comprennent du phosphate.

4. Procédé selon une ou plusieurs des revendications 1 à 3, **caractérisé en ce que** le tampon de liaison comprend additionnellement des chélateurs, tels que EDTA.

5. Procédé selon une ou plusieurs des revendications 1 à 4, **caractérisé en ce que** la phase solide est constituée par des particules de magnétite présentant un diamètre de 0,01 à 2 µm et une aire de surface spécifique de 1 - 100 m²/g.

6. Kit pour l'isolation d'ARN au moyen du procédé selon une ou plusieurs des revendications 1 à 5, comprenant au moins une phase solide de magnétite et un tampon de liaison présentant une concentration en GTC supérieure à 3 mol/l.

7. Kit selon la revendication 6, **caractérisé en ce que** le tampon de liaison comprend au moins entre 4 et 8 mol/l de GTC et entre 5 et 200 mmol/l d'EDTA.

8. Kit selon la revendication 6 ou 7, **caractérisé en ce que** le kit comprend additionnellement un ou plusieurs des constituants qui suivent:
- un tampon d'élution
- un tampon de lavage
- une solution de sel de phosphate.
